(19)

Europäisches
Patentamt

European
Patent Office

Office européen
des brevets

(11) **EP 4 725 423 A1**

(12) **EUROPEAN PATENT APPLICATION**

(43) Date of publication:
**15.04.2026 Bulletin 2026/16**

(21) Application number: **25208182.3**

(22) Date of filing: **13.10.2025**

(51) International Patent Classification (IPC):
**A61B 6/50** (2024.01)   **A61B 6/00** (2024.01)
**G06T 7/11** (2017.01)   **G16H 50/20** (2018.01)

(52) Cooperative Patent Classification (CPC):
**A61B 6/503; A61B 6/5217; G06T 7/11;
G16H 30/40; G16H 50/20;** A61B 6/032; A61B 6/481;
G06T 2207/10081; G06T 2207/30048

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **14.10.2024 US 202418914346**

(71) Applicants:
• **Siemens Healthineers AG
91301 Forchheim (DE)**

• **The Methodist Hospital
Houston, TX 77030 (US)**

(72) Inventors:
• **JACOB, Athira Jane
Princeton, NJ 08540-6632 (US)**
• **RAPAKA, Saikiran
Princeton, NJ 08540-6632 (US)**
• **CHANG, Su Min
Houston, TX 77030 (US)**

(74) Representative: **HKW Intellectual Property PartG
mbB
Theresienhöhe 12
80339 München (DE)**

(54) **EJECTION FRACTION FROM NON-CONTRAST COMPUTED TOMOGRAPHY**

(57)     For segmentation (310) and/or ejection fraction determination (320), photon-counting detection (100) for CECT in ES is used to create (110) virtual NCCT for machine training (130). The lack of NCCT data at ES for training (130) segmentation is overcome by use of the virtual NCCT. Since the segmentation (310) is trained (130) on non-contrast imaging at ES, accurate ES segmentation (310) from NCCT may be provided. The EF may be calculated (320) using NCCT.

```
300 ──┐
       ┌─────────────────────────┐
       │  Acquire NCCT Data at ES │
       └─────────────────────────┘
                    │
                    ▼
310 ──┐
       ┌─────────────────────────┐
       │   Segment NCCT at ES     │
       └─────────────────────────┘
                    │
                    ▼
320 ──┐
       ┌─────────────────────────┐
       │      Determine EF        │
       └─────────────────────────┘
                    │
                    ▼
330 ──┐
       ┌─────────────────────────┐
       │     Display Image        │
       └─────────────────────────┘
```

FIG. 3

EP 4 725 423 A1

**Description**

BACKGROUND

The present embodiments relate to non-contrast computed tomography (NCCT). Cardiac chamber volume may be determined from NCCT scans. Volumetry from NCCT scans has the advantages of reduced risk and cost from contrast administration, lower radiation doses as compared to contrast enhanced computed tomography (CECT).

In one approach for volume determination from NCCT, a deep learning (DL) model is trained to segment cardiac chambers using paired NCCT and contrast enhanced CT (CECT) scans used for calcium scoring. The ground truth contours were created by transferring annotations from the CECT scans to the NCCT scans. However, these scans are acquired at the end diastolic (ED) phase, so the volume segmentation is for the ED phase. NCCT data is not typically available for the end-systolic (ES) phase, limiting the training and the segmentation from NCCT to ED.

Ejection fraction (EF) quantification relies on ED and ES measurements of the volume. EF quantification is performed using CECT scans, not NCCT, resulting in the risks, costs, and doses associated with adding contrast agent to the patient. NCCT determination of EF may have potential for screening and triage of cardiac patients.

SUMMARY

The invention is defined by the attached set of claims. Further details of the disclosed method, devices and system are described below, which are helpful for understanding the claimed invention.

By way of introduction, the preferred embodiments described below include methods, systems, and non-transitory computer readable media for segmentation and/or ejection fraction determination. Photon-counting detection for CECT in ES is used to create virtual NCCT for machine training. The lack of NCCT data at ES for training segmentation is overcome by use of the virtual NCCT. Since the segmentation is trained on non-contrast imaging at ES, accurate ES segmentation from NCCT may be provided. The EF may be calculated using NCCT.

In a first aspect, a method is provided for determining ejection fraction by a computed tomography system. The computed tomography system acquires NCCT data representing a heart of a patient. An image processor segments the heart in the NCCT data. A value for the ejection fraction is determined from the heart as segmented in the non-contrast computed tomography data. The value of the ejection fraction is displayed.

In a second aspect, a method is provided for machine training a model for segmentation from non-contrast computed tomography scans. Contrast computed tomography data is obtained by a photon-counting computed tomography detector. The contrast computed tomography data represents patients at end-systole. Response from the added contrast is removed from the contrast computed tomography data. The removal creates virtual non-contrast computed tomography data representing the patients at end-systole. The model is machine trained with input from the virtual non-contrast computed tomography data and output of segmentations of heart chambers at end-systole. The machine-trained model is stored.

In a third aspect, a system is provided for segmentation in computed tomography. A computed tomography detector is configured to detect, at end-systole, data from a scan of a patient free of added contrast agent. An image processor is configured to reconstruct, by computed tomography, a representation of a heart of the patient from the data and to segment, with a deep-learned network, a chamber at end-systole from the representation. A display is configured to display the segmented chamber at end-systole or a value derived from the segmented chamber.

Any one or more of the aspects or concepts summarized above or in the Illustrative Embodiments below may be used alone or in combination. The aspects or concepts described for one Illustrative Embodiment or aspect may be used in other embodiments or aspects. The aspects or concepts described for a method or system may be used in others of a system, method, or non-transitory computer readable storage medium.

The present invention is defined by the following claims, and nothing in this section should be taken as a limitation on those claims. Further aspects and advantages of the invention are discussed below in conjunction with the preferred embodiments and may be later claimed independently or in combination.

BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.

Figure 1 is a flow chart of an example implementation of a method for machine training for segmentation from NCCT at ES;

Figure 2 illustrates example training and inference for NCCT segmentation at ES to calculate EF;

Figure 3 is a flow chart diagram of an example implementation of a method for determining EF from segmentation of NCCT at ES;

Figure 4 is a block diagram of an example implementation of a CT system for NCCT based segmentation at ES and/or for EF determination;

Figure 5 shows an exemplary artificial neural network that may be used to implement one or more embodiments; and

Figure 6 shows a convolutional neural network that may be used to implement one or more embodiments.

DETAILED DESCRIPTION OF THE DRAWINGS AND PRESENTLY PREFERRED EMBODIMENTS

While NCCT scans can be prospectively acquired at ES phase, this is not common practice. It is challenging to gather enough paired data in the ES phase to machine train for segmentation of heart volume at ES. To overcome this lack of training data, machine training for segmentation at ES uses virtual non-contrast (VNC) scans from photon-counting CT (PCCT). PCCT has been used for CECT scans at ES phases. Due to the use of PCCT, the response from the added contrast in CECT may be removed, creating the VNC. The VNC is used machine train to segment cardiac chambers at the ES phase. A large amount of ES specific VNC data with registered ground truth (GT) contours from CECT is provided. The machine-learned model may receive newly acquired NCCT data at ES to accurately segment. This could reduce the need for contrasted CT scans.

EF may be calculated from NCCT. Since accurate segmentation from NCCT at ES is provided, EF may be accurately calculated despite the lack of NCCT data at ES. EF can be used for risk stratification for heart failure, for monitoring, progression, and/or treatment guidance without risks, costs, and/or doses associated with CECT.

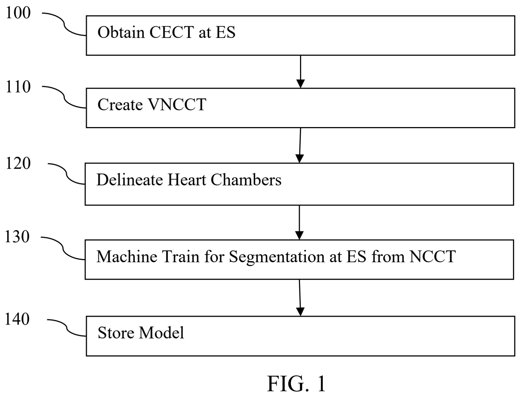

Figure 1 shows one implementation of a flow chart of a method for machine training a model for segmentation, which may be used for EF determination, from NCCT scans. Since the main gap in volumetry from NCCT scans is volume quantification in ES frames, a deep-learning (DL) network or another machine learning model is trained to segment gated cardiac NCCT at ES. A large database of ES NCCT scans is created as VNC scans from photon counting systems at the ES phase. Ground truth contours may be directly derived from the CECT scans and superimposed on the VNC scans with known spatial registration. This captures the left ventricle and/or other chambers at the ES phase, enabling the DL network to implicitly model the contracted heart shape at ES from NCCT scans.

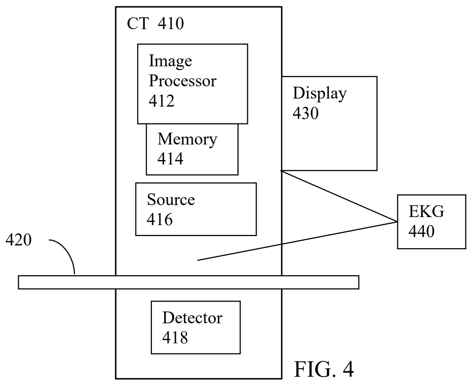

The method of Figure 1 is implemented by a computer, workstation, server, or another image processor. A memory is used to store the machine-learned model (e.g., DL network) and training data. The image processor trains the model using the training data. One or more photon-counting CT systems, such as shown in Figure 4, may be used to obtain CT data by scanning. A database for radiology may be used to obtain CT data from previous scanning.

Additional, different, or fewer acts may be performed. For example, act 140 is not performed. As another example, acts for application of the trained model are provided.

The acts are performed in the order shown (numerical or top-to-bottom) or a different order. For example, act 120 is performed before or after act 110.

In act 100, CECT or other contrast-based computed tomography data is obtained. CECT or contrast CT will be used as an example herein. Contrast agent, such as iodine, is ingested by or injected into patients. A CT scan is performed on the patient. The resulting data represents, in part, the attenuation of x-rays or density caused by the added contrast agent. The resulting data also represents attenuation of x-rays caused by or density of tissue and/or other objects in the patients.

The data is obtained from a database, such as a radiology database of scans or imaging of past patients. In another approach, the data is obtained by scanning patients. A processor obtains, with or without user selection, and a memory stores the obtained contrast CT data. The processor may mine or search for the contrast CT. Alternatively, the user identifies a database or file for the processor to use.

The contrast CT data is scan data, such as raw x-ray readings from a detector. In other implementations, the contrast CT data is a tomographically reconstructed representation of a three-dimensional object, such as the heart or a chamber of the heart. In yet other implementations, the contrast CT data is one or more images generated from a tomographically reconstructed representation. Data responsive to tissue and contrast agent at any point or stage of the CT imaging pipeline may be used as the contrast CT data.

The contrast CT data is gated. The CT system or scanner is triggered to detect at a particular point in time. Alternatively, CT scan data from the particular point in time is selected. The gating results in contrast CT representing the contrast and patient a given time or times. For example, electrocardiogram tracing is used to gate to a given phase or phases of the heart cycle. ES, ED, and/or other phases may be used for gating. For example, contrast CT data is separately acquired at ES and ED, providing contrast CT data representing the patient at these two phases.

For machine training, many samples are acquired as training data. Hundreds, thousands, or more samples of contrast CT at ES and/or ED are obtained.

Since the model is to be machine trained to segment from NCCT, the contrast CT data is to be converted to NCCT data. The CT system or detector used to detect the contrast CT data has or is a photon-counting detector. The photon-counting CT detector counts photon interactions by energy level, so provides spectral information. The deposited energy of each interaction of x-rays with the detector is tracked, providing an approximate energy spectrum. By binning energy at different levels, each counted photon is assigned to a respective bin. Each pixel of the detector provides an energy histogram. The energy histogram or another energy spectrum representation allows for distinguishing material composition. This capability allows for conversion of the contrast CT to NCCT data. Contrast CT data may be more widely available, even using photon-counting, than NCCT.

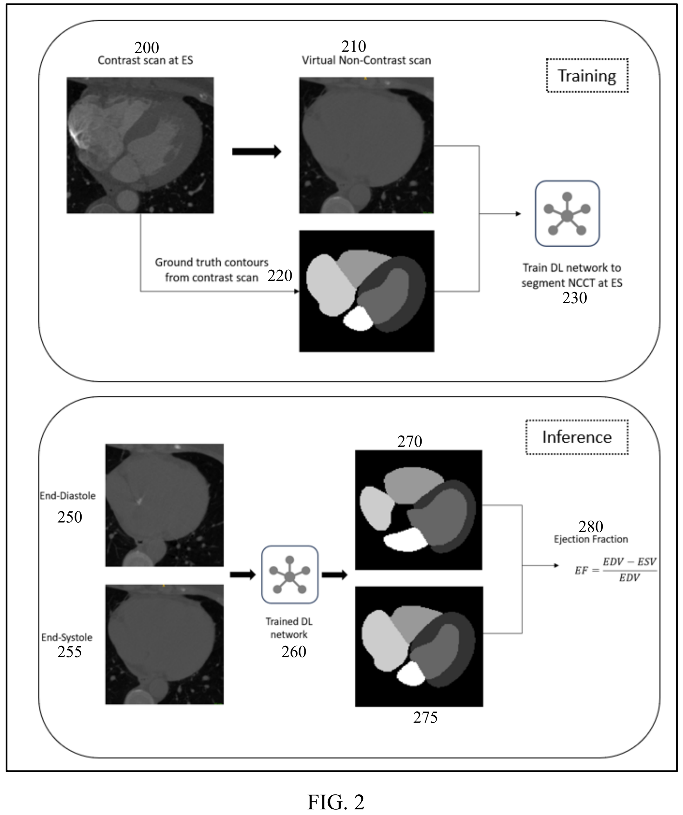

Figure 2 shows an example in the training portion. A contrast scan 200 is acquired at ES.

In act 110, the processor removes response from the added contrast from the contrast CT data. The energy histogram or energy spectrum from photon counting is used to remove the contribution of the added contrast agent. The counts as a function of energy of the added contrast agent are removed from the contrast CT data. The added contrast agent has a known energy response. This energy response is removed, such as subtracting out the counts as a function of energy of the added contrast agent. The remaining counts represent the attenuation from or density of tissue and/or other objects without the added contrast agent. By removing for each pixel at which the added contrast agent is detected, the resulting CT data is VNC CT.

The contrast CT data is from ES and/or ED. Once the contribution from the added contrast agent is removed, the VNC CT data representing patient tissue and/or other non-added contrast agent objects is provided. The VNC CT data for ES and/or ED is provided for each, most, or many of the samples used as training data.

In the example of Figure 2, the contrast scan 200 has the response from iodine removed. This creates the VNC scan 210.

In act 120, the processor delineates one or more heart chambers. The contrast CT data is segmented. A programmed segmentation, machine-learned or artificial intelligence segmentation, or expert curated segmentation is provided. The segmentation identifies the voxels within the chamber, the surface of the chamber, or both. In one implementation, the left ventricle is segmented.

The delineation of the chamber or chambers is used as a ground truth in machine training. Since the VNC CT to be used as input samples of NCCT and the delineation are from the contrast CT, the ground truth segmentation of the chamber or chambers is registered with the input samples. The delineation or segmentation is used as the ground truth to compare with delineation or segmentation output by the machine-learning model during training.

In the example of Figure 2, the contrast scan 200 is segmented. Four different chambers are identified in the segmentation 220. Fewer chambers, such as just one, may be segmented.

In act 130, the processor machine trains a model with input from the VNC CT data and output of segmentations of one or more heart chambers. The many samples of created VNC CT are used as sample inputs, and the corresponding ground truths are compared to output chambers for optimizing the model through machine training.

The machine learning is performed for ES. The training data representing ES are used to train the model. A different model of the same or different structure is trained for ED. Alternatively, one joint model is trained to segment for both ES and ED inputs using training data from both phases.

The model architecture is defined. The user inputs an architecture of the model to be used in machine training. Different building blocks, such as neural network layers, activation functions, nodes, and/or other groupings, are linked together. Learnable parameters may be defined, including limits on the parameters. Fixed parameters or set values may be included or not in the definition of the architecture. Connections and weights may be defined. A default or pre-programmed model may be selected. The model may be formed by alteration of another model, such as provided in pre-training.

The model is a neural network, such as a convolutional neural network or a fully connected neural network. In one implementation, the model is a U-net, encoder-decoder, variational autoencoder, conditional variational autoencoder, an image-to-image network or another network. The model or network may be configured for deep learning, so is a deep-learning network. For example, an encoder and a decoder based on convolutional neural network is used with one input for NCCT or VNC and an output for segmentation. Support vector machines or other machine learning models may be used.

An image processor, such as a workstation or server, machine trains the defined model, such as machine training the image-to-image network. The machine training uses training data. Hundreds or thousands of samples as training data are obtained, such as many samples of the VNC scan 210 and ground truth segmentation 220 of Figure 2. The image processor machine trains the deep-learning network 230 using the training data.

The machine training uses the samples to learn values of learnable parameters of the defined model. Using optimization, such as Adam, the values are determined by iteratively varying the values to find a combination that provides output best matching the ground truth given the range of inputs in the training data. In one embodiment, deep learning of a defined neural network is performed. Other types of machine learning may be used. Supervised learning (e.g., using ground truth for loss from the generator) or semi-supervised learning may be used.

In act 140, the image processor stores the machine-trained model. After training, the defined architecture, values of the learnable parameters, and values of set parameters are stored. Any format may be used.

The machine-trained model is stored in a memory, such as a memory local to the image processor. Additionally, or alternatively, the machine-trained model is transmitted or delivered to one or more (e.g., multiple) memories, such as at different healthcare facilities and/or CT scanners.

The values of the stored or trained machine-learned model are fixed. The values of the learnable parameters are set for application to CT data for one or more patients. The values do not change during application for a patient. The machine-learned model may be retrained or updated using additional training data.

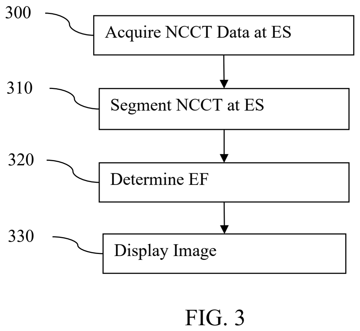

Figure 3 is a flow chart of one implementation of a method for segmenting from NCCT and/or determining EF by a CT system. The machine-learned model trained as described in Figures 1 and 2 is applied in an inference stage. The machine-learned model trained to segment NCCT at ES is applied. The stored machine-learned model segments one or more chambers for a patient at ES. The resulting segmentation may be used for medical diagnostic quantification, such as determining EF from heart chamber volumes at ES and ED.

The method of Figure 3 is implemented by an image processor, a CT system, a server, a workstation, and/or another component or system. For example, the CT system performs act 300. The CT system is a photon-counting CT system or is a conventional CT system (i.e., uses an energy-integrating detector). An image processor, such as the image processor of the CT system or a different image processor, performs acts 310 and 320. A display of the CT system, the image processor, or another display performs act 330. Other devices or components may be used instead or in addition to the imaging systems and/or processors.

Additional, different, or fewer acts may be performed. For example, act 330 is not performed. As another example, act 320 is not performed, such as where segmentation at ES is performed without determining the EF.

The acts are performed in the order shown (numerical or top-to-bottom) or a different order.

In act 300, the CT system acquires NCCT data representing a heart of a patient. The entire heart or a portion of the heart is represented. A CT scan of the patient is performed by the CT system (scanner). Other scanners to measure the attenuation or density at different locations or along lines through the patient may be used. Alternatively, the attenuation or density information is acquired from memory, such as information from a previously performed CT scan.

The CT scan is of a volume of the patient. The CT scan provides measures of attenuation of the x-ray energy or density at different locations, such as voxels, within the patient. The attenuations or densities of the voxels are computed by tomography from a sequence of x-ray scans from different angles through the patient. The resulting CT intensity data represents voxels of the CT scan volume.

The CT data is a measure in Hounsfield units. This represents the density of the tissue at different locations or attenuation of the x-rays. The CT data may be converted or mapped to attenuation values. For example, a bilinear transformation is performed using a look-up table or a machine-learned network.

The CT data represents the patient free of or without added contrast agent. Rather than a CECT scan, a NCCT scan is performed. The added cost, risks, and/or doses for CECT and the corresponding added contrast agent is avoided. The CT data is acquired free of added contrast medium in the patient. Free of added contrast agent or medium is in the context of contrast added for CT imaging. Other contrast agent, such as for ultrasound, may or may not be in the patient for acquiring the NCCT data.

The NCCT data is acquired at ES. Using gating, NCCT data representing the heart of the patient at ES is acquired. For EF determination, NCCT data representing the heart of the patient at ED is also acquired using gating. Gating may trigger the scan or may be used to select the NCCT data to be used.

In the example of Figure 2 under inference, two separate gated NCCT scans 250, 255 are taken or performed, one during ES of the heart and one during ED of the heart. Each of the scans 250, 255 results in NCCT data.

In act 310, the image processor segments the heart in the NCCT data. One or more chambers represented in the NCCT data are segmented. The locations of the surface and/or the voxels belonging to the chamber or chambers are located.

The segmentation is performed for both ED and ES. The NCCT data at ED is segmented, and the NCCT data at ES is separately segmented. Joint segmentation may be provided, such as where information from both ES and ED is used in the segmentation at ES and ED.

In the example of Figure 2, the trained deep-learning network 260 (e.g., network 230 as deep learned) generates the segmentation 270 of four chambers of the heart of the patient at ED and the segmentation 275 of four chambers of the heart of the patient at ES. While one network 260 is shown, separate networks 260 may be provided for ES and ED segmentation. During inference, the image processor runs the trained model(s) 260 on NCCT scans 250, 255 from ED and ES to segment the chambers, providing segmentations 270, 275.

The segmentation is performed by the image processor applying the machine-learned model (e.g., image-to-image network). The NCCT data is input to the machine-learned model, which outputs the segmentation of one or more heart chambers in response. In one implementation, the machine-learned model is a convolutional neural network as described for Figure 1. Other neural networks or models may be used.

The machine-learned model operates with NCCT data as the input. Rather than inputting CECT data, the model was trained with VNC data so that NCCT data may be input, at least for ES. Many NCCT data samples may be available for ED, so a model may be trained using NCCT data for ED. For ES, fewer samples may be available, so VNC CT data was used in the training. VNC CT data was created using CECT photon-counting scans with a photon-counting detector. The VNC CT data was formed by removal of spectrum information for the added contrast agent from the photon-counting CT scans.

The machine-learned model outputs the segmentation as labels for spatial locations belonging to a chamber. Binary labeling may be used, such as 1 for voxels of the chamber and 0 for voxels outside the chamber. In another implementation, the machine-learned model outputs a probabilistic segmentation. A probability of each voxel being part of the chamber is output. For voxels along a boundary of the chamber, less than 100% and more than 0% probability may be used. Voxels adjacent to or spaced from the boundary may have less than 100% and more than 0% probability of being part of the chamber.

While Figure 2 shows the chambers in different gray scale in two dimensions, the segmentations 270, 275 represent each chamber in three-dimensions. Different gray scale is used to distinguish different chambers. Other separation, such as different labeling or annotation, may be used to distinguish different chambers. The segmentations 270, 275 may be for only one chamber, such as the left ventricle. The segmentations 270, 275, using the known size of the voxels from the NCCT scan 250, 255, provides a volume of the heart chamber at ES and ED.

In act 320, the image processor determines a value for the EF from the heart as segmented in the NCCT data. The image processor calculates the volumes of the chamber (e.g., left ventricle) at ES and ED. The segmentations are used to calculate a value for the EF. A difference in volume from ES to ED is calculated. The EF is the stroke volume divided by the ED volume. The stroke volume is a difference or change in volume of volume at ES subtracted from volume at ED. Figure 2 shows an example calculation of EF 280 from the segmentations 270, 275 of the left ventricle. For a percentage, the EF is multiplied by 100.

The value of EF is calculated from NCCT data. Other information may be used to assist in segmentation and/or volume calculation. In one implementation, the only imaging or scan data representing the heart used to determine EF is the NCCT data.

Where the segmentation for ES and/or ED is probabilistic, the value of EF may be determined as a range. The probabilistic segmentation provides a range of volumes for ES and/or ED. These ranges of volumes are used to determine a range of EF.

Other diagnostic quantifications may be calculated instead or in addition to EF using the ES segmentation from NCCT data. For example, stroke volume, cardiac output, or another volume-based measurement of the heart may be determined.

In act 330, a display displays the value of the EF. The numerical value is a singular value or a range (e.g., from probabilistic segmentation). An image is generated with the value being part of the image. The value may be an annotation on a CT image of the heart, included in a displayed medical record, provided in a graph or chart, and/or otherwise presented to the physician for diagnosis.

The obtained EF as the numerical value can be used as a clinical measurement. In another implementation, the image processor applies one or more thresholds to detect normal or abnormal or to detect along a scale. The value may be displayed as the results of the comparison, such as normal or abnormal or a marker or color code along a scale. The value indicates whether further investigation of heart condition for the patient is warranted.

The image with the value may include one or more CT images. For example, a multi-planar reconstruction with a three-dimensional rendered view is displayed. As anther example, a two-dimensional image of a standard heart view is displayed. Images from ES and ED may be displayed at a same time or alone. The image is rendered, such as three-dimensional rendered, from the voxels of the tomographic reconstruction to a two-dimensional display image.

Figure 4 is a block diagram of one implementation of a system for segmentation and/or EF determination in CT. The system includes a CT scanner (CT system) 410. The system implements the method of Figure 1, the method of Figure 3, and/or another method. Where used to obtain training data in the method of Figure 1, the CT scanner 410 is a photon-counting CT system with a photon-counting detector 418. Where used to acquire NCCT data in the method of Figure 3, the CT scanner 410 is a photon-counting CT system or a conventional CT system (i.e., uses an energy-integrating detector 418).

The system includes the CT scanner 410 and an electrocardiogram (EKG or ECG) 440. Additional, different, or fewer components may be provided. In an example, the system includes a computer network for communications between components.

The EKG 440 includes electrodes, processor, and a communications interface. The EKG 440 detects electrical signals from the heart of the patient while on the bed 420. The signals are processed to identify the heart trace or heart cycle. This heart cycle and/or triggers (e.g., ED and ES triggers) derived from the heart cycle are output to the CT scanner 410 (e.g., image processor 412, another controller, and/or the display 430).

The CT scanner 410 includes an image processor 412, memory 414, X-ray source 416, detector 418, patient bed 420, and display 430. The image processor 412, memory 414, and/or display 430 are part of the CT scanner 410 or are separate (e.g., a computer or workstation). Additional, different, or fewer components may be provided. For example, the system is a computer without the source 416, detector 418, and/or bed 420, instead relying on data acquired by a separate scanner. As another example, the CT scanner 410 includes power supplies, communications interfaces, and user interface systems.

The CT scanner 410 includes the x-ray source 416 and opposing detector 418 mounted in a gantry. The CT scanner 410 is an x-ray scanner configured to obtain attenuation or density data (e.g., measures of tissue density in Hounsfield units) for a patient volume. The gantry moves the source 416 and detector 418 about the patient for scanning. The image processor 412 or a different processor tomographically computes the attenuation of the x-rays and/or density at different voxels within the scan volume. Any now known or later developed CT scanner 410 may be used. Other x-ray scanners, such as a CT-like C-arm scanner, may be used.

The CT scanner 410 is configured to detect data from a scan of the patient. The scan is configured (e.g., by energy or power of the source 416, speed and/or trajectory of the gantry, and/or duration of the scan and/or source activation) to scan a patient on the bed 420 where the patient is free of added contrast agent. The CT scanner 410 is configured for a NCCT scan.

The CT scanner 410 is configured to gate the detector 418 and/or scan to detect at ES and/or ED. The NCCT scan is gated to the heart cycle.

The image processor 412 is a general processor, digital signal processor, graphics processing unit, application specific integrated circuit, field programmable gate array, artificial intelligence processor, digital circuit, analog circuit, combinations thereof, or other now known or later developed device for controlling the CT scanner 410, tomography, application of a machine-learned model, rendering of an image, segmentation, and/or display image generation. The processor 412 is a single device, a plurality of devices, or a network. For more than one device, parallel or sequential division of processing may be used. Different devices making up the processor 412 may perform different functions, such as one processor for handling segmentation and another processor for calculating EF and/or image generation. In one embodiment, the processor 412 is a control processor or other processor of the CT system 410. In other embodiments, the processor 412 is part of a separate workstation, server, or computer.

The processor 412 operates pursuant to stored instructions to perform various acts described herein. The processor 412 is configured by software, design, firmware, and/or hardware to perform any or all the acts of Figures 1 and/or 3.

For training, the image processor 412 is configured to access the training data and machine-learned model in the memory 414 and machine train.

For application or inference, the processor 412 is configured to reconstruct, by computed tomography, a representation of a heart of the patient from an NCCT scan and to segment, with a deep-learned network, a chamber at end-systole from the representation.

The deep-learned network is an image-to-image machine-learned network in one implementation. The deep-learned network was previously trained from training data of VNC data at ES created from photon-counting CT scans of subjects of the patient with added contrast agent. The deep-learned network was trained from the training data where the VNC CT data was created by removal of spectrum information for the added contrast agent from the photon-counting CT scans.

In one implementation, the image processor 412 applies the deep-learned network to output a segmented chamber. The NCCT data is input to the deep-learned network, which outputs the segmented chamber in response to the input. The input NCCT data is from a given phase of the heart cycle, such as ES, so the output represents the segmented chamber at that phase. In a further implementation, the deep-learned network is configured by past training to output the segmented chamber as a probabilistic segmentation.

The image processor 412 is configured to calculate a value as an EF from a volume of the segmented chamber at ES and a volume of the segmented chamber at ED. The same or different deep-learned models are used to segment at ES and ED. The image processor 412 calculates the volume at each phase from the respective segmentations. The image processor 412 calculates the value of EF from the volumes. Where the segmentation is probabilistic, the image processor 412 may calculate the value as a range of EF. Thresholds may be applied so that the value represents a classification based on the EF.

The display 430 is configured by the image processor 412 (e.g., the image processor 412 loads an image into a display plane buffer of the display 430). The display 430 is configured to display the segmented chamber at ES and/or a value (e.g., value of EF) derived from the segmented chamber. The display is a CRT, LCD, plasma screen, projector, printer, or other output device for showing an image.

The memory 414 stores CT data, training data, NCCT data, segmentations, the deep-learned model or network, a value for EF, thresholds, heart phase label, and/or other information used or generated in the methods of Figure 1 and/or Figure 3.

The memory 414 is additionally or alternatively a non-transitory computer readable storage medium with processing instructions. The memory 414 stores data representing instructions executable by the programmed processor 412, such as instructions for segmentation and/or EF calculation or instructions for machine training.

The instructions for implementing the processes, methods and/or techniques discussed herein are provided on non-transitory computer-readable storage media or memories, such as a cache, buffer, RAM, removable media, hard drive or other computer readable storage media. Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone or in combination. Likewise, processing strategies may include multiprocessing, multitasking, parallel processing and the like. In one embodiment, the instructions are stored on a removable media device for reading by local or remote systems. In other embodiments, the instructions are stored in a remote location for transfer through a computer network or over telephone lines. In yet other embodiments, the instructions are stored within a given computer, CPU, GPU, or system.

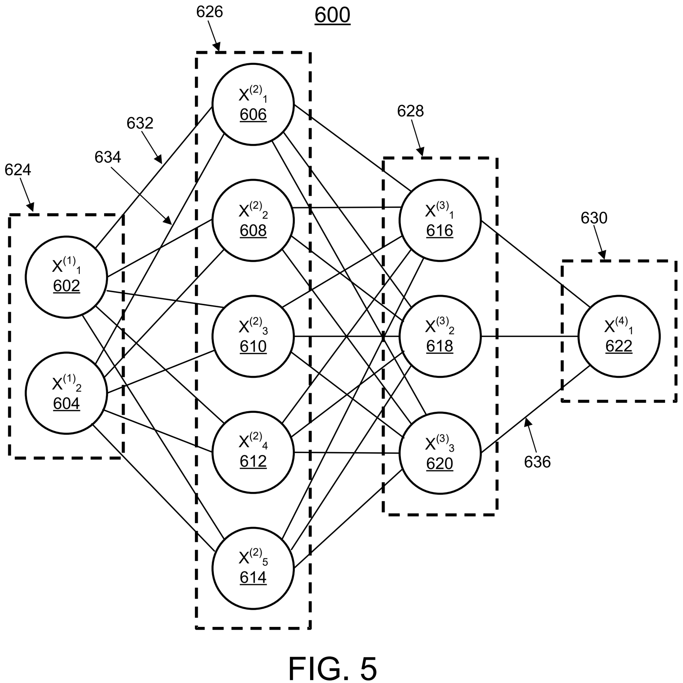

Figure 5 shows an embodiment of an artificial neural network 600, in accordance with one or more embodiments (e.g., network 230 to be trained or network 260 as trained). Alternative terms for "artificial neural network" are "neural network", "artificial neural net" or "neural net". Machine learning networks described herein, such as, e.g., the one or more machine learning based networks utilized at step 130 of Figure 1, the networks 230, 260 of Figure 2, the network used in act 310 of Figure 3, or any other machine learning network described herein may be implemented using artificial neural network 600.

The artificial neural network 600 comprises nodes 602-622 and edges 632, 634, ..., 636, wherein each edge 632, 634, ..., 636 is a directed connection from a first node 602-622 to a second node 602-622. In general, the first node 602-622 and the second node 602-622 are different nodes 602-622, it is also possible that the first node 602-622 and the second node 602-622 are identical. For example, in Figure 5, the edge 632 is a directed connection from the node 602 to the node 606, and the edge 634 is a directed connection from the node 604 to the node 606. An edge 632, 634, ..., 636 from a first node 602-622 to a second node 602-622 is also denoted as "ingoing edge" for the second node 602-622 and as "outgoing edge" for the first node 602-622.

In this embodiment, the nodes 602-622 of the artificial neural network 600 can be arranged in layers 624-630, wherein the layers can comprise an intrinsic order introduced by the edges 632, 634, ..., 636 between the nodes 602-622. In particular, edges 632, 634, ..., 636 can exist only between neighboring layers of nodes. In the embodiment shown in Figure 5, there is an input layer 624 comprising only nodes 602 and 604 without an incoming edge, an output layer 630 comprising only node 622 without outgoing edges, and hidden layers 626, 628 in-between the input layer 624 and the output layer 630. In general, the number of hidden layers 626, 628 can be chosen arbitrarily. The number of nodes 602 and 604 within the input layer 624 usually relates to the number of input values of the neural network 600, and the number of nodes 622 within the output layer 630 usually relates to the number of output values of the neural network 600.

In particular, a (real) number can be assigned as a value to every node 602-622 of the neural network 600. Here, $x^{(n)}_i$ denotes the value of the i-th node 602-622 of the n-th layer 624-630. The values of the nodes 602-622 of the input layer 624 are equivalent to the input values of the neural network 600, the value of the node 622 of the output layer 630 is equivalent to the output value of the neural network 600. Furthermore, each edge 632, 634, ..., 636 can comprise a weight being a real number, in particular, the weight is a real number within the interval [-1, 1] or within the interval [0, 1]. Here, $w^{(m,n)}_{i,j}$ denotes the weight of the edge between the i-th node 602-622 of the m-th layer 624-630 and the j-th node 602-622 of the n-th layer 624-630. Furthermore, the abbreviation $w^{(n)}_{i,j}$ is defined for the weight $w^{(n,n+1)}_{i,j}$.

In particular, to calculate the output values of the neural network 600, the input values are propagated through the neural network. In particular, the values of the nodes 602-622 of the (n+1)-th layer 624-630 can be calculated based on the values of the nodes 602-622 of the n-th layer 624-630 by $x_j^{(n+1)} = f\left(\sum_i x_i^{(n)} \cdot w_{i,j}^{(n)}\right)$. Herein, the function f is a transfer function (another term is "activation function"). Known transfer functions are step functions, sigmoid function (e.g. the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 624 are given by the input of the neural network 600, wherein values of the first hidden layer 626 can be calculated based on the values of the input layer 624 of the neural network, wherein values of the second hidden layer 628 can be calculated based in the values of the first hidden layer 626, etc.

In order to set the values $w^{(m,n)}_{i,j}$ for the edges, the neural network 600 has to be trained using training data. In particular, training data comprises training input data and training output data (denoted as $t_i$). For a training step, the neural network 600 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 600 (backpropagation algorithm). In particular, the weights are changed according to:

$$w'^{(n)}_{i,j} = w^{(n)}_{i,j} - \gamma \cdot \delta^{(n)}_{j} \cdot x^{(n)}_{i}$$

wherein y is a learning rate, and the numbers $\delta^{(n)}_{j}$ can be recursively calculated as:

$$\delta^{(n)}_{j} = \left( \sum_k \delta^{(n+1)}_{k} \cdot w^{(n+1)}_{j,k} \right) \cdot f' \left( \sum_i x^{(n)}_{i} \cdot w^{(n)}_{i,j} \right)$$

based on $\delta^{(n+1)}_{j}$, if the (n+1)-th layer is not the output layer, and

$$\delta^{(n)}_{j} = \left( x^{(n+1)}_{k} - t^{(n+1)}_{j} \right) \cdot f' \left( \sum_i x^{(n)}_{i} \cdot w^{(n)}_{i,j} \right)$$

if the (n+1)-th layer is the output layer 630, wherein f' is the first derivative of the activation function, and $y^{(n+1)}_{j}$ is the comparison training value for the j-th node of the output layer 630.

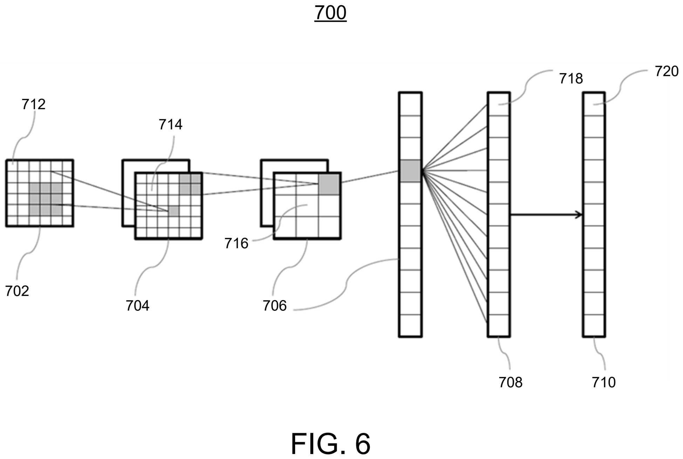

Figure 6 shows a convolutional neural network 700, in accordance with one or more embodiments. Machine learning networks described herein, such as, e.g., the one or more machine learning based networks utilized at step 130 of Figure 1, the networks 230, 260 of Figure 2, the network used in act 310 of Figure 3, or any other machine learning network described herein may be implemented using convolutional neural network 700.

In the embodiment shown in Figure 6, the convolutional neural network comprises 700 an input layer 702, a convolutional layer 704, a pooling layer 706, a fully connected layer 708, and an output layer 710. Alternatively, the convolutional neural network 700 can comprise several convolutional layers 704, several pooling layers 706, and several fully connected layers 708, as well as other types of layers. The order of the layers can be chosen arbitrarily, usually fully connected layers 708 are used as the last layers before the output layer 710.

In particular, within a convolutional neural network 700, the nodes 712-720 of one layer 702-710 can be considered to be arranged as a d-dimensional matrix or as a d-dimensional image. In particular, in the two-dimensional case the value of the node 712-720 indexed with i and j in the n-th layer 702-710 can be denoted as $x^{(n)}_{[i,j]}$. However, the arrangement of the nodes 712-720 of one layer 702-710 does not have an effect on the calculations executed within the convolutional neural network 700 as such, since these are given solely by the structure and the weights of the edges.

In particular, a convolutional layer 704 is characterized by the structure and the weights of the incoming edges forming a convolution operation based on a certain number of kernels. In particular, the structure and the weights of the incoming edges are chosen such that the values $x^{(n)}_{k}$ of the nodes 714 of the convolutional layer 704 are calculated as a convolution $x^{(n)}_{k} = K_k * x^{(n-1)}$ based on the values $x^{(n-1)}$ of the nodes 712 of the preceding layer 702, where the convolution * is defined in the two-dimensional case as

$$x^{(n)}_{k}[i,j] = \left( K_k * x^{(n-1)} \right)[i,j] = \sum_{i'} \sum_{j'} K_k[i',j'] \cdot x^{(n-1)}[i-i', j-j'] .$$

Here the k-th kernel $K_k$ is a d-dimensional matrix (in this embodiment a two-dimensional matrix), which is usually small compared to the number of nodes 712-718 (e.g. a 3x3 matrix, or a 5x5 matrix). In particular, this implies that the weights of the incoming edges are not independent, but chosen such that they produce said convolution equation. In particular, for a kernel being a 3x3 matrix, there are only 9 independent weights (each entry of the kernel matrix corresponding to one independent weight), irrespectively of the number of nodes 712-720 in the respective layer 702-710. In particular, for a convolutional layer 704, the number of nodes 714 in the convolutional layer is equivalent to the number of nodes 712 in the preceding layer 702 multiplied with the number of kernels.

If the nodes 712 of the preceding layer 702 are arranged as a d-dimensional matrix, using a plurality of kernels can be interpreted as adding a further dimension (denoted as "depth" dimension), so that the nodes 714 of the convolutional layer 704 are arranged as a (d+1)-dimensional matrix. If the nodes 712 of the preceding layer 702 are already arranged as a (d+1)-dimensional matrix comprising a depth dimension, using a plurality of kernels can be interpreted as expanding along the depth dimension, so that the nodes 714 of the convolutional layer 704 are arranged also as a (d+1)-dimensional matrix, wherein the size of the (d+1)-dimensional matrix with respect to the depth dimension is by a factor of the number of kernels larger than in the preceding layer 702.

The advantage of using convolutional layers 704 is that spatially local correlation of the input data can exploited by enforcing a local connectivity pattern between nodes of adjacent layers, in particular by each node being connected to only a small region of the nodes of the preceding layer.

In embodiment shown in Figure 6, the input layer 702 comprises 36 nodes 712, arranged as a two-dimensional 6x6 matrix. The convolutional layer 704 comprises 72 nodes 714, arranged as two two-dimensional 6x6 matrices, each of the two matrices being the result of a convolution of the values of the input layer with a kernel. Equivalently, the nodes 714 of the convolutional layer 704 can be interpreted as arranges as a three-dimensional 6x6x2 matrix, wherein the last dimension is the depth dimension.

A pooling layer 706 can be characterized by the structure and the weights of the incoming edges and the activation function of its nodes 716 forming a pooling operation based on a non-linear pooling function f. For example, in the two dimensional case the values $x^{(n)}$ of the nodes 716 of the pooling layer 706 can be calculated based on the values $x^{(n-1)}$ of the nodes 714 of the preceding layer 704 as

$$x^{(n)}[i,j] = f(x^{(n-1)}[id_1, jd_2], ..., x^{(n-1)}[id_1+d_1-1, jd_2+d_2-1])$$

In other words, by using a pooling layer 706, the number of nodes 714, 716 can be reduced, by replacing a number d1·d2 of neighboring nodes 714 in the preceding layer 704 with a single node 716 being calculated as a function of the values of said number of neighboring nodes in the pooling layer. In particular, the pooling function f can be the max-function, the average or the L2-Norm. In particular, for a pooling layer 706 the weights of the incoming edges are fixed and are not modified by training.

The advantage of using a pooling layer 706 is that the number of nodes 714, 716 and the number of parameters is reduced. This leads to the amount of computation in the network being reduced and to a control of overfitting.

In the embodiment shown in Figure 6, the pooling layer 706 is a max-pooling, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

A fully-connected layer 708 can be characterized by the fact that a majority, in particular, all edges between nodes 716 of the previous layer 706 and the nodes 718 of the fully-connected layer 708 are present, and wherein the weight of each of the edges can be adjusted individually.

In this embodiment, the nodes 716 of the preceding layer 706 of the fully-connected layer 708 are displayed both as two-dimensional matrices, and additionally as non-related nodes (indicated as a line of nodes, wherein the number of nodes was reduced for a better presentability). In this embodiment, the number of nodes 718 in the fully connected layer 708 is equal to the number of nodes 716 in the preceding layer 706. Alternatively, the number of nodes 716, 718 can differ.

Furthermore, in this embodiment, the values of the nodes 720 of the output layer 710 are determined by applying the Softmax function onto the values of the nodes 718 of the preceding layer 708. By applying the Softmax function, the sum the values of all nodes 720 of the output layer 710 is 1, and all values of all nodes 720 of the output layer are real numbers between 0 and 1.

A convolutional neural network 700 can also comprise a ReLU (rectified linear units) layer or activation layers with non-linear transfer functions. In particular, the number of nodes and the structure of the nodes contained in a ReLU layer is equivalent to the number of nodes and the structure of the nodes contained in the preceding layer. In particular, the value of each node in the ReLU layer is calculated by applying a rectifying function to the value of the corresponding node of the preceding layer.

The input and output of different convolutional neural network blocks can be wired using summation (residual / dense neural networks), element-wise multiplication (attention) or other differentiable operators. Therefore, the convolutional neural network architecture can be nested rather than being sequential if the whole pipeline is differentiable.

In particular, convolutional neural networks 700 can be trained based on the backpropagation algorithm. For preventing overfitting, methods of regularization can be used, e.g. dropout of nodes 712-720, stochastic pooling, use of artificial data, weight decay based on the L1 or the L2 norm, or max norm constraints. Different loss functions can be combined for training the same neural network to reflect the joint training objectives. A subset of the neural network parameters can be excluded from optimization to retain the weights pretrained on another datasets.

Listed below are various Illustrative Embodiments. The Illustrative Embodiments summarize different combinations of aspects. Other combinations of any of the aspects with any other one or more of the aspects may be provided. Aspects from one type (e.g., method or system) may be used in another type (system or method).

Illustrative Embodiment 1. A method for determining ejection fraction by a computed tomography system, the method comprising: acquiring, by the computed tomography system, non-contrast computed tomography data representing a heart of a patient; segmenting, by an image processor, the heart in the non-contrast computed tomography data; determining a value for the ejection fraction from the heart as segmented in the non-contrast computed tomography data;

and displaying the value for the ejection fraction.

Illustrative Embodiment 2. The method of Illustrative Embodiment 1, wherein acquiring comprises acquiring the non-contrast computed tomography data representing the heart at both end-systole and end-diastole, wherein segmenting comprises segmenting the heart at both end-systole and end-diastole, and wherein determining comprises determining the value from a difference in volumes of the heart at end-systole and end-diastole.

Illustrative Embodiment 3. The method of any of Illustrative Embodiments 1-2, wherein acquiring comprises acquiring free of added contrast medium in the patient, and wherein determining comprises determining from the non-contrast computed tomography data as the only image data representing the heart used in the determination.

Illustrative Embodiment 4. The method of any of Illustrative Embodiments 1-3, wherein segmenting comprises segmenting by input of the non-contrast computed tomography data to a machine-learned model, the machine-learned model outputting a segmentation of a heart chamber, and wherein determining comprises determining the value from the segmentation.

Illustrative Embodiment 5. The method of Illustrative Embodiment 4, wherein segmenting comprises segmenting by the machine-learned model, the machine-learned model comprising a convolutional neural network.

Illustrative Embodiment 6. The method of any of Illustrative Embodiments 4-5, wherein segmenting comprises segmenting by the machine-learned model, the machine-learned model having been trained from training data of virtual non-contrast data at end systole created from photon-counting computed tomography scans of subjects with added contrast agent.

Illustrative Embodiment 7. The method of Illustrative Embodiment 6, wherein segmenting comprises segmenting by the machine-learned model, the machine-learned model having been trained from the training data where the virtual non-contrast data was created by removal of spectrum information for the added contrast agent from the photon-counting computed tomography scans.

Illustrative Embodiment 8. The method of any of Illustrative Embodiments 4-7, wherein segmenting comprises segmenting by the machine-learned model, the machine-learned model outputting the segmentation as a probabilistic segmentation, wherein determining comprises determining the value as a range from the probabilistic segmentation, and wherein displaying the value comprises displaying the range.

Illustrative Embodiment 9. A method for machine training a model for segmentation from non-contrast computed tomography scans, the method comprising: obtaining contrast computed tomography data by a photon-counting computed tomography detector, the contrast computed tomography data representing patients at end-systole; removing response from the added contrast from the contrast computed tomography data, the removal creating virtual non-contrast computed tomography data representing the patients at end-systole; machine training the model with input from the virtual non-contrast computed tomography data and output of segmentations of heart chambers at end-systole; and storing the machine-trained model.

Illustrative Embodiment 10. The method of Illustrative Embodiment 9, wherein removing comprises removing counts as a function of energy of the added contrast agent from the contrast computed tomography data.

Illustrative Embodiment 11. The method of any of Illustrative Embodiments 9-10, further comprising delineating the heart chambers in the contrast computed tomography data, the delineations comprising ground truth for the machine training to output the segmentations.

Illustrative Embodiment 12. A system for segmentation in computed tomography, the system comprising: a computed tomography detector configured to detect, at end-systole, data from a scan of a patient free of added contrast agent; an image processor configured to reconstruct, by computed tomography, a representation of a heart of the patient from the data and to segment, with a deep-learned network, a chamber at end-systole from the representation; and a display configured to display the segmented chamber at end-systole or a value derived from the segmented chamber.

Illustrative Embodiment 13. The system of Illustrative Embodiment 12, wherein the deep-learned network comprises an image-to-image machine-learned network.

Illustrative Embodiment 14. The system of any of Illustrative Embodiments 12-13, further comprising an electrocardiogram, wherein the computed tomography detector is gated by the electrocardiogram to detect at end-systole.

Illustrative Embodiment 15. The system of any of Illustrative Embodiments 12-14, wherein the deep-learned network is configured to output the segmented chamber as a probabilistic segmentation.

Illustrative Embodiment 16. The system of Illustrative Embodiment 15, wherein the image processor is configured to calculate the value as a range of an ejection fraction using the probabilistic segmentation.

Illustrative Embodiment 17. The system of any of Illustrative Embodiments 12-16, wherein the image processor is configured to calculate the value as an ejection fraction from a volume of the segmented chamber at end-systole.

Illustrative Embodiment 18. The system of any of Illustrative Embodiments 12-17, wherein the deep-learned network was trained from training data of virtual non-contrast data at the end systole created from photon-counting computed tomography scans of subjects with added contrast agent.

Illustrative Embodiment 19. The system of Illustrative Embodiment 18, wherein the deep-learned network was trained from the training data where the virtual non-contrast data was created by removal of spectrum information for the added contrast agent from the photon-counting computed tomography scans.

While the invention has been described above by reference to various embodiments, it should be understood that many changes and modifications can be made without departing from the scope of the invention. It is therefore intended that the foregoing detailed description be regarded as illustrative rather than limiting, and that it be understood that it is the following claims, including all equivalents, that are intended to define the scope of this invention.

**Claims**

1.   A method for determining (320) ejection fraction by a computed tomography system (410), the method comprising:

   acquiring (300), by the computed tomography system (410), non-contrast computed tomography data representing a heart of a patient;
   segmenting (310), by an image processor (412), the heart in the non-contrast computed tomography data;
   determining (320) a value for the ejection fraction from the heart as segmented in the non-contrast computed tomography data; and
   displaying (330) the value for the ejection fraction.

2.   The method of claim 1,

   wherein acquiring (300) comprises acquiring (300) the non-contrast computed tomography data representing the heart at both end-systole and end-diastole, segmenting (310) comprises segmenting (310) the heart at both end-systole and end-diastole, and determining (320) comprises determining (320) the value from a difference in volumes of the heart at end-systole and end-diastole; and/or
   wherein acquiring (300) comprises acquiring (300) free of added contrast medium in the patient, and determining (320) comprises determining (320) from the non-contrast computed tomography data as the only image data representing the heart used in the determination.

3.   The method of claim 1 or 2, wherein segmenting (310) comprises segmenting (310) by input of the non-contrast computed tomography data to a machine-learned model, in particular segmenting (310) by a machine-learned model comprising a convolutional neural network (260), the machine-learned model outputting a segmentation of a heart chamber, and wherein determining (320) comprises determining (320) the value from the segmentation.

4.   The method of claim 3, wherein segmenting (310) comprises segmenting (310) by the machine-learned model, the machine-learned model having been trained from training data of virtual non-contrast data at end systole created from photon-counting computed tomography scans of subjects with added contrast agent.

5.   The method of claim 4, wherein segmenting (310) comprises segmenting (310) by the machine-learned model, the machine-learned model having been trained from the training data where the virtual non-contrast data was created by removal of spectrum information for the added contrast agent from the photon-counting computed tomography scans.

6.   The method of any one of claims 3 - 5, wherein segmenting (310) comprises segmenting by the machine-learned model, the machine-learned model outputting the segmentation as a probabilistic segmentation, wherein determining (320) comprises determining (320) the value as a range from the probabilistic segmentation, and wherein displaying (330) the value comprises displaying (330) the range.

7.   A method for machine training (130) a model for segmentation from non-contrast computed tomography scans, the method comprising:

   obtaining (100) contrast computed tomography data by a photon-counting computed tomography detector (418), the contrast computed tomography data representing patients at end-systole;
   removing (110) response from the added contrast from the contrast computed tomography data, the removal creating virtual non-contrast computed tomography data representing the patients at end-systole;
   machine training (130) the model with input from the virtual non-contrast computed tomography data and output of segmentations of heart chambers at end-systole; and
   storing (140) the machine-trained model.

8.   The method of claim 7, wherein removing (110) comprises removing (110) counts as a function of energy of the added contrast agent from the contrast computed tomography data.

9. The method of claim 7 or 8, further comprising delineating the heart chambers in the contrast computed tomography data, the delineations comprising ground truth for the machine training (130) to output the segmentations.

10. A system for segmentation in computed tomography, the system comprising:

a computed tomography detector (418) configured to detect, at end-systole, data from a scan of a patient free of added contrast agent;
an image processor (412) configured to reconstruct, by computed tomography, a representation of a heart of the patient from the data and to segment, with a deep-learned network (260), in particular with a deep-learned network (260) comprising an image-to-image machine-learned network (260), a chamber at end-systole from the representation; and
a display (430) configured to display the segmented chamber at end-systole or a value derived from the segmented chamber.

11. The system of claim 10, further comprising an electrocardiogram (440), wherein the computed tomography detector (418) is gated by the electrocardiogram (440) to detect at end-systole.

12. The system of claim 10 or 11, wherein the deep-learned network (260) is configured to output the segmented chamber as a probabilistic segmentation.

13. The system of claim 12, wherein the image processor (412) is configured to calculate the value as a range of an ejection fraction using the probabilistic segmentation.

14. The system of any one of claims 10 - 13, wherein the image processor (412) is configured to calculate the value as an ejection fraction from a volume of the segmented chamber at end-systole.

15. The system of any one of claims 10 - 14, wherein the deep-learned network (260) was trained from training data of virtual non-contrast data at the end systole created from photon-counting computed tomography scans of subjects with added contrast agent, in particular wherein the deep-learned network (260) was trained from the training data where the virtual non-contrast data was created by removal of spectrum information for the added contrast agent from the photon-counting computed tomography scans.

100 — Obtain CECT at ES

110 — Create VNCCT

120 — Delineate Heart Chambers

130 — Machine Train for Segmentation at ES from NCCT

140 — Store Model

FIG. 1

FIG. 2

300 — Acquire NCCT Data at ES

310 — Segment NCCT at ES

320 — Determine EF

330 — Display Image

FIG. 3

CT 410

Image
Processor
412

Memory
414

Source
416

420

Detector
418

Display
430

EKG
440

FIG. 4

FIG. 5

700

FIG. 6

Europäisches
Patentamt

European
Patent Office

Office européen
des brevets

# EUROPEAN SEARCH REPORT

**Application Number**

EP 25 20 8182

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/120095 A1 (NAGHAVI MORTEZA [US] ET AL) 11 April 2024 (2024-04-11) | 1-3,10, 11,14 | INV.<br>A61B6/50 |
| Y | * paragraph [0027] * | 4,5,7-9, | A61B6/00 |
|   | * paragraph [0052] * | 12,15 | G06T7/11 |
| A | * paragraph [0062] * | 6,13 | G16H50/20 |
|   | * paragraph [0066] - paragraph [0070] * | | |
|   | * paragraph [0075] - paragraph [0090] * | | |
|   | & NAGHAVI MORTEZA ET AL: "AI-enabled cardiac chambers volumetry in coronary artery calcium scans (AI-CACTM) predicts heart failure and outperforms NT-proBNP: The multi-ethnic study of Atherosclerosis", JOURNAL OF CARDIOVASCULAR COMPUTED TOMOGRAPHY, ELSEVIER, AMSTERDAM, NL, vol. 18, no. 4, 24 April 2024 (2024-04-24) , pages 392-400, XP087554929, ISSN: 1934-5925, DOI: 10.1016/J.JCCT.2024.04.006 [retrieved on 2024-04-24] | | |
|   | * 2.3 AI-CAC; figures * | | **TECHNICAL FIELDS SEARCHED (IPC)**<br><br>A61B |
|   | ----- | | G16H |
| Y | STEFFEN BRUNS ET AL: "Deep Learning from Dual-Energy Information for Whole-Heart Segmentation in Dual-Energy and Single-Energy Non-Contrast-Enhanced Cardiac CT", ARXIV.ORG, CORNELL UNIVERSITY LIBRARY, 201 OLIN LIBRARY CORNELL UNIVERSITY ITHACA, NY 14853, 10 August 2020 (2020-08-10), XP081737593, DOI: 10.1002/MP.14451 | 4,5,7-9, 15 | G06T |
| A | * the whole document * | 6,13 | |
|   | ----- | | |
| Y | US 2018/182101 A1 (PETERSEN PETER KERSTEN [US] ET AL) 28 June 2018 (2018-06-28) | 12 | |
| A | * paragraph [0020] - paragraph [0023] * | 6,13 | |
|   | * paragraph [0042] - paragraph [0063] * | | |
|   | ----- | | |

The present search report has been drawn up for all claims

| Place of search | Date of completion of the search | Examiner |
|---|---|---|
| Munich | 4 February 2026 | Strubel, Christine |

EPO FORM 1503 03.82 (P04C01)

# EP 4 725 423 A1

## ANNEX TO THE EUROPEAN SEARCH REPORT
## ON EUROPEAN PATENT APPLICATION NO.

EP 25 20 8182

This annex lists the patent family members relating to the patent documents cited in the above-mentioned European search report.
The members are as contained in the European Patent Office EDP file on
The European Patent Office is in no way liable for these particulars which are merely given for the purpose of information.

04-02-2026

| Patent document cited in search report | Publication date | Patent family member(s) | | Publication date |
|---|---|---|---|---|
| US 2024120095 A1 | 11-04-2024 | NONE | | |
| US 2018182101 A1 | 28-06-2018 | EP | 3559907 A1 | 30-10-2019 |
| | | EP | 4145391 A1 | 08-03-2023 |
| | | JP | 7134962 B2 | 12-09-2022 |
| | | JP | 2020503603 A | 30-01-2020 |
| | | US | 2018182101 A1 | 28-06-2018 |
| | | US | 2020184646 A1 | 11-06-2020 |
| | | US | 2022383495 A1 | 01-12-2022 |
| | | WO | 2018119358 A1 | 28-06-2018 |

EPO FORM P0459

For more details about this annex : see Official Journal of the European Patent Office, No. 12/82